# EUROPÄISCHE PATENTSCHRIFT

(11) **EP 3 935 045 B1**
(45) Veröffentlichungstag und Bekanntmachung des Hinweises auf die Patenterteilung: **21.05.2025**
(21) Anmeldenummer: 20706521.0
(22) Anmeldetag: 28.02.2020
(51) Int. Cl.: C07D 213/73, C07D 471/04, A61K 31/4375, A61P 9/00

(54) **SYNTHESE VON 4-AMINO-5-METHYL-1H-PYRIDIN-2(1H)-ON (ZWISCHENVERBINDUNG DER SYNTHESE DES MR ANTAGONSITEN FINERENONE) AUS 2-CHLORO-5-METHYL-4-NITRO-PYRIDINE-1-OXID ÜBER DIE ZWISCHENVERBINDUNG 2-CHLORO-5-METHYL-4-PYRIDINAMIN**
SYNTHESIS OF 4-AMINO-5-METHYL-1H-PYRIDIN-2(1H)-ONE (INTERMEDIATE IN THE SYNTHESIS OF THE MR ANTAGONIST FINERENONE) FROM 2-CHLORO-5-METHYL-4-PYRIDINAMINE VIA THE INTERMEDIATE 2-CHLORO-5-METHYL-4-PYRIDINEAMINE
SYTHÈSE DE 4-AMINO-5-MÉTHYL-1H-PYRIDIN-2(1H)-ONE (INTERMÉDIAIRE DE LA SYNTHÈSE DU ANTAGONIST DE MR FINÉRÉNONE) À PARTIR DE 2-CHLORO-5-METHYL-4-PYRIDINAMINE VIA LE INTERMÉDIARE 2-CHLORO-5-METHYL-4-PYRIDINEAMINE

(30) Priorität: 05.03.2019 EP 19160904
(43) Veröffentlichungstag der Anmeldung: 12.01.2022
(73) Patentinhaber: Bayer Aktiengesellschaft, 51373 Leverkusen (DE); Bayer Pharma Aktiengesellschaft, 13353 Berlin (DE)
(72) Erfinder: PLATZEK, Johannes, 12621 Berlin (DE); LOVIS, Kai, 40215 Düsseldorf (DE)
(74) Vertreter: BIP Patents
(86) Internationale Anmeldenummer: PCT/EP2020/055292
(87) Internationale Veröffentlichungsnummer: WO 2020/178175

(56) Entgegenhaltungen:
- CN-A- 1 311 185
- CN-A- 103 193 704

## Beschreibung

Die vorliegende Erfindung betrifft ein neues und verbessertes Verfahren zur Herstellung von 4-Amino-5-methylpyridon der Formel (I) 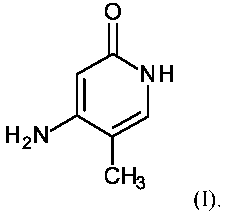 4-Amino-5-methylpyridon der Formel (I) wird durch Umsetzten von Chlor-Methyl-Amino-Pyridin (2) mit KOH in Methanol im Autoklaven bei erhöhter Temperatur hergestellt.

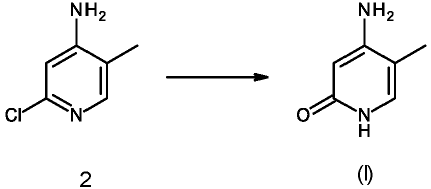

Die Erfindung betrifft auch ein Verfahren zur Herstellung von Chlor-Methyl-Amino-Pyridin (2)

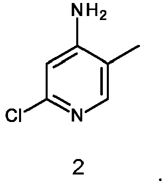

Hierbei wird das Nitro-N-Oxid der Formel (3) 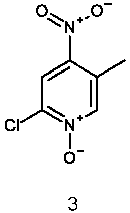 an einem Platinkatalysator hydriert, wobei Chlor-Methyl-Amino-Pyridin (2) erhalten wird. Mit dem erfindungsgemäßen Verfahren gelingt es ausgehend vom Nitro-N-Oxid (3) über zwei 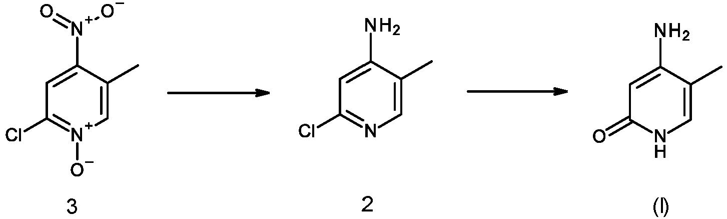 chemische Stufen die Zielverbindung (I) mit einer Gesamtausbeute von 84 % in einer hohen Reinheit (> 99 %) herzustellen.

Die Verbindung der Formel (I) ist ein Schlüssel-Intermediat für die Herstellung von Finerenone (II):

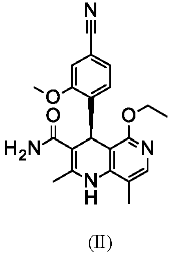

Finerenone (II) wirkt als nicht steroidaler Antagonist des Mineralcorticoid Rezeptors und kann als Mittel zur Prophylaxe und /oder Behandlung von kardiovaskulären und renalen Erkrankungen wie beispielsweise Herzinsuffizienzen und diabetische Nephropathie eingesetzt werden.

CN 103 193 704 A offenbart ein Verfahren zur Herstellung von 4-Amino-5-methyl-2(1H)-pyridinon durch Umsetzung von 2-Chloro-5-methyl-4-pyridinamin in Ethylenglykol sowie ein Verfahren zur Herstellung von 2-Chloro-5-methyl-4-pyridinamin durch Reduktion von 2-Chloro-5-methyl-4-nitro-pyridine-1-oxid mit Eisenpulver in HCI. CN 1 311 185 A offenbart ein Verfahren zur Herstellung von 2-Chloro-5-methyl-4-pyridinamin durch Reduktion von 2-Chloro-5-methyl-4-nitro-pyridine-1-oxid durch Hydrierung an einem Raney-Nickel Katalysator.

Die Verbindung der Formel (II) und deren Herstellungsprozess sind in der WO 2008/104306 und ChemMedChem 2012, 7, 1385 sowie in WO 2016/016287 A1 (Bayer Pharma AG) beschrieben, wobei in beiden Publikationen eine ausführliche Diskussion der Forschungs-Synthese offenbart ist. Nachteilig an der dort beschriebenen Synthese ist die Tatsache, dass sich diese Synthese nicht für ein weiteres großtechnisches Verfahren eignet, da viele Schritte in sehr hoher Verdünnung, mit sehr hohen Reagenz-Überschüssen und damit zu einer relativ geringen Gesamtausbeute verlaufen.

Es bestand daher der Bedarf nach einer großtechnisch praktikablen Synthese, die das Verfahrensintermediat der Verbindung der Formel (I) reproduzierbar in hoher Gesamtausbeute, niedrigen Gestehungskosten und hoher Reinheit liefert und allen regulatorischen Anforderungen entspricht, um die klinischen Prüfungen mit Wirkstoff zu beliefern und für eine spätere behördliche Einreichung verwendet zu werden.

Die Herstellung von Verbindung (I) ist in Synthesis S. 765 (1984) beschrieben (Beispiel 3c). Ausgehend von Malonsäurechlorid und Propionitril wird in 40 % der Theorie Ausbeute das Chlorpyridin-Hydrochlorid erhalten, was anschließend direkt einer Hydrierung mit Pd/C unterzogen wird: 86 % der Theorie. Die Gesamtausbeute über beide Stufen 34,4 % der Theorie

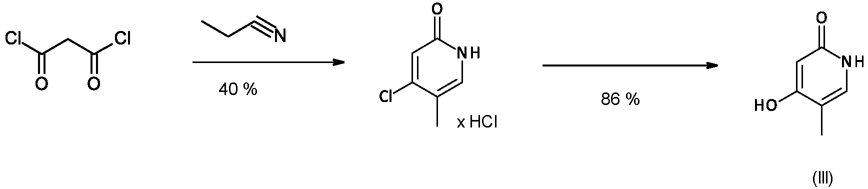

Ausgehend vom Hydroxypridon (III), welches in Beispiel 1c der Synthesis-Publikation beschrieben ist, wird in siedendem Benzylamin (IV) zur Verbindung (V) umgesetzt. Anschließend wird die Benzylgruppe in Verbindung (V) durch katalytische Hydrierung über Palladium/Kohle ab hydriert. Die Gesamtausbeute über beide Stufen beträgt 62,4 % der Theorie:

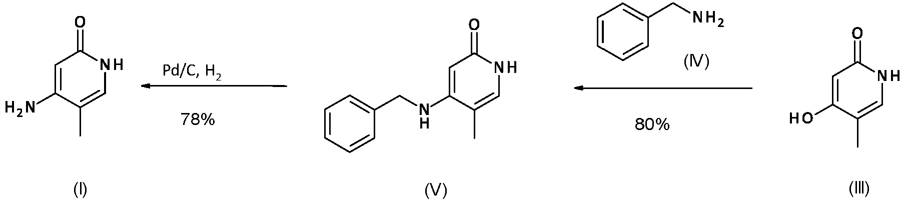

Nachteil des Verfahrens ist die Verwendung von einem sehr großen Überschuss an Benzylamin, für 30 mmol der Verbindung der Formel (III), werden 30 ml (275,2 mmol) verwendet, das ist ein 9,17 facher Überschuss bezogen auf Verbindung (III). Das Recycling von überschüssigem Benzylamin ist aufwendig und mit erheblichen Kosten verbunden. Die Reaktion wird in siedendem Benzylamin (185° C) durchgeführt, die Reaktionszeit beträgt 36 Stunden. Solch hohe Temperaturen sind in Standard-Rührwerken nicht durchführbar und erfordern spezielles technisches Equipment. Beim Nachkochen der Vorschrift fiel besonders ein Nebenprodukt (VI) auf, das auf Spuren von Palladium aus der Vorstufe zu (III) zurückzuführen ist:

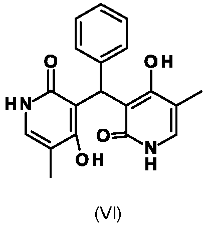

Unter den drastischen Reaktionsbedingungen kommt es zu einer Dehydrierung zum Benzlimin, welches dann zum Benzaldehyd zerfällt (es wird Wasser während der Reaktion gebildet), der Benzaldehyd kondensiert mit Verbindung der Formel (III) zu Verbindung der Formel (VI). Dieses Nebenprodukt entsteht besonders beim Up-Scalen (bis > 10%) der Ansätze und schleppt sich bis zur Verbindung der Formel (I) mit durch. Zur Aufarbeitung werden nach Abkühlung der Reaktionslösung auf Raumtemperatur, die ausgefallenen Kristalle mit Methylethylketon und o-Dichlorbenzol gewaschen und anschließend aus o-Dichlorbenzol umkristallisiert. Auch hier wäre es vorteilhaft auf chlorierte Lösungsmittel zu verzichten und umweltfreundlichere Varianten verfolgen.

Die nachfolgende Debenzylierung läuft in Eisessig, 10 mmol in 200 ml, das sind 2,14 g Verbindung (V) in 200 ml. Dieses entspricht einem 93,45 fachem Überschuss, das heißt für 1 kg (V) bräuchte man 93,45 L Essigsäure. Dieses sind riesige Überschüsse, die für ein technisches Verfahren nicht in Frage kommen. Des weiteren werden für die Umsetzung von 10 mmol 600 mg Pd-Katalysator auf Kohle (10% und 30%) eingesetzt. Das heißt, um 1 kg Verbindung (V) zu debenzylieren, wären 280 g Katalysator notwendig. Auch das ist aus technischer und ökonomischer Sicht nicht sinnvoll. Zur Aufarbeitung wird vom Katalysator abfiltriert und das Filtrat zur Trockene eingedampft, mit Toluol werden Reste Essigsäure herausgeschleppt und der Rückstand in Aceton oder Methylethylketon aufgenommen und abfiltriert. Dieser Vorgang ist beim Upscaling technisch nicht realisierbar, da in Rührwerken nicht zur Trockene eingedampft wird. Außerdem werden drei verschiedene Lösungsmittel für die Isolierung benötigt. Das stark gefärbte Reaktionsprodukt wird anschließend noch per Chromatographie gereinigt (Dichlormethan/MeOH 1: 1), auch das möchte man für einen großtechnischen Prozess nach Möglichkeit vermeiden. Die Gesamtausbeute über 4 chemische Stufen, ausgehend von Malonsäuredichlorid beträgt 21,4 % der Theorie

Aufgabe der vorliegenden Erfindung war die Entwicklung einer alternativen Synthese für das Verfahrensintermediat 4-Amino-5-methylpyridon als Zwischenprodukt für die Herstellung von Verbindungen der Formel (II), Finerenone, insbesondere ein Verfahren welches sich großtechnisch gut durchführen lässt, kostengünstig ist und große Lösungsmittelüberschüsse vermeidet und mit Reagenzien auskommt die umweltfreundlicher sind.

Mit der vorliegenden Erfindung wurde eine sehr effiziente Synthese gefunden, die es erlaubt die oben genannten Nachteile zu umgehen. Ausgehend von dem in der Literatur bekannten Chlor-Methyl-Amino-Pyridin (2) 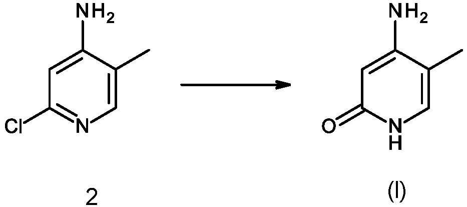 gelangt man zur Ziel-Verbindung (I), indem man Verbindung (2) im Autoklaven bei erhöhter Temperatur entweder unter sauren oder basischen Bedingungen umsetzt. In Tetrahedron 55 (1999), S. 11985 ist eine ähnliche Umsetzung mit NaOH in Methanol beschrieben. Die Aufreinigung erfolgt über eine Chromatographie. Leider ergab die Umsetzung von Verbindung 2 unter diesen Bedingungen ein Gemisch aus Zielverbindung (I) und dem 2-Methylether (7):

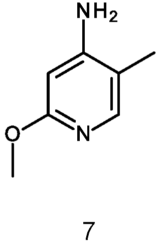

Dieses Gemisch konnte nur chromatographisch aufgetrennt werden.

Überraschender Weise läuft die Umsetzung zur Zielverbindung sehr glatt, wenn man Anstelle von Natriumhydroxid, Kaliumhydroxid (KOH) verwendet. Dann läuft die Umsetzung auch in Methanol als Lösungsmittel, ohne dass der Methylether (7) als Nebenprodukt erhalten wird. Vorzugsweise arbeitet man in reinem Methanol als Lösungsmittel, jedoch kann auch in wässrigem Methanol gearbeitet werden. Die Reaktion wird in einem Autoklaven bei Temperaturen von 160 bis 200 °C, bevorzugt bei 180°C durchgeführt. Reaktionszeiten betragen 15-48 Stunden, je nach gewählter Temperatur, das heißt inter alia sind die Reaktionszeiten bei höheren Temperaturen eher kürzer. Zur Aufarbeitung wird mit einer Mineralsäure,zum Beispiel Salzsäure, Schwefelsäure oder Phosphorsäure, bevorzugt Salzsäure neutralisiert (pH ca. 7) und dann weitgehend eingeengt und durch Zugabe zu Ethanol Wasser azeotrop entfernt. Zum Schluss wird in Methanol umdestilliert und die Salze abfiltriert. Man engt weitgehend ein und destilliert auf Wasser um. Zur Umkristallisation wird auf ca. dreifache Volumenmenge (bezogen auf Ausgangsmaterial 2) eingeengt. Man kühlt auf 0°C ab, isoliert das Produkt, beispielsweise durch

Filtration, wäscht gegebenenfalls mit wenig kaltem Wasser nach und trocknet unter Vakuum bei erhöhter Temperatur (30 - 70°C).

Gegenstand der vorliegenden Erfindung ist daher ein Verfahren zur Herstellung des Verfahrensintermediats 4-Amino-5 methylpyridon der Formel (I) dadurch gekennzeichnet dass man Chlor-Methyl-Amino-Pyridin (2) im Autoklaven mit KOH in Methanol umsetzt.

In einer bevorzugten Ausführungsform arbeitet man bei Temperaturen von 160°C bis 200°C insbesondere bei 180°C.

Ein weiterer Gegenstand der vorliegenden Erfindung betrifft ein Verfahren zur Herstellung des Verfahrensintermediats 4-Amino-5-methylpyridon der Formel (I) dadurch gekennzeichnet, dass man zuerst das Intermediat Nitro-N-Oxid (3) an einem Platinkatalysator hydriert und anschließend das erhaltene Intermediat Chlor-Methyl-Amino-Pyridin (2) im Autoklaven mit Kaliumhydroxid (KOH) in Methanol umsetzt.

In einer Ausführungsform der Erfindung wird zunächst Chlor-Methyl-Amino-Pyridin (2) hergestellt und kann dann in dem erfindungsgemäßen Verfahren eingesetzt werden:

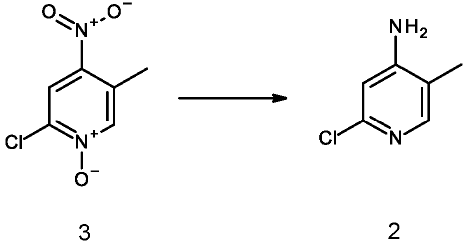

Hierbei werden unter Verwendung eines Pt-Katalysators gleichzeitig die Nitrogruppe und das Nitro-N-Oxid reduziert. Eine derartige katalytische Hydrierungs-Reaktion ist bei einem solchen Substitutionsmuster am Pyridin noch nicht in der Literatur beschrieben.

Die Herstellung von Verbindung 2 ist literaturbekannt (WO 2005/100342 A1), auch die Des-methyl-Verbindung von 2 ist bekannt und wird nach ähnlichen Methoden hergestellt (Tetrahedron 55 (1999), S. 1195), jedoch sind die dort beschriebenen Methoden schwer in den technisch größeren Maßstab zu übertragen (Up-Scaling), da teilweise mit elementaren Zink oder Eisen unter sauren Bedingungen, bevorzugt Essigsäure gearbeitet wird. Auch die Verwendung von Raney-Nickel als Hydrier-Katalysator ist möglich, jedoch ist das Up-Scaling problematisch, da Raney-Nickel-Abfälle extrem pyrophor sind. Des Weiteren ist die technische Durchführung sicherheitstechnisch eine Herausforderung, da sich die Reaktion auf Grund der starken Exothermie nicht gut steuern lässt. Des Weiteren gestaltet sich die Aufarbeitung als sehr schwierig und aufwendig und liefert obendrein eine Menge an zu entsorgenden Metall-Salz-Abfall, was bei der Herstellung derartiger Produkte im Tonnen-Maßstab nicht unerheblich ist.

Eine häufig in auftretende Nebenreaktion bei der Reduktion von 2-Chlor substituierten Pyridin-Derivate ist die gleichzeitige Reduktion des Chloratoms zum Wasserstoff-Rest (Verbindung 4).

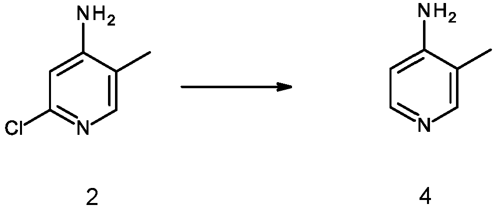

Diese Nebenreaktion wird in diesem neuen erfinderischen Verfahren nicht, oder nur in untergeordnetem Maßstab (<< 1%) beobachtet und war so für den Fachmann überraschend und nicht zu erwarten. Die Reaktion wird bevorzugt in protischen Lösungsmitteln zum Beispiel Alkohole wie Ethanol, Methanol, Isopropanol, n-Propanol, n-Butanol durchgeführt. Es können aber Lösungsmittel wie THF, Dioxan, 2-Methyl-THF verwendet werden. In manchen Fällen kann der Zusatz von Wasser vorteilhaft sein.

Als Katalysator wird bevorzugt ein Platin-haltiger Katalysator verwendet. Folgende Platin Katalysatoren können verwendet werden:

| |
|---|
| 0,8% Pt + 0,6% Mo on Carbon powder |
| 1 % Pt + 2% V on Carbon powder |
| 0,5 % Pt + 0,3 % Mo on Carbon powder |

Besonders bevorzugt wird 0,8% Pt + 0,6% Mo on Carbon powder (von der BASF) verwendet. Der Wasserstoff-Druck bei der Hydrierung sollte zwischen 2 bis7 bar, bevorzugt 2 bis 5, besonders bevorzugt bei 3 bar liegen. Die Temperatur sollte zwischen 20 bis 50°C, bevorzugt aber zwischen 25 bis 30°C, besonders bevorzugt bei 30°C liegen. Die Reaktionszeit beträgt 10 bis 30 Stunden, bevorzugt 18 bis 22 h.

Zur Isolierung wird vom Katalysator abfiltriert und die Lösung weitgehend eingeengt und auf das Lösungsmittel der Folgereaktion, bevorzugt Methanol umdestilliert. Vorteilhafterweise wird das Rohprodukt direkt in die Folgestufe eingesetzt. Die Reaktion verläuft quantitativ.

Die Herstellung vom Nitro-N-Oxid (3) ist literaturbekannt, wie zum Beispiel in Heterocycles, Vol. 78, No 11, 2009, S 2811 oder in WO 2005/100342 A1 beschrieben.

In Heterocycles, Vol. 78, No 11, 2009 sind folgende Ausbeuten für die Herstellung von Verbindung 3 beschrieben: Gesamtausbeute 64% der Theorie über 2 Stufen.

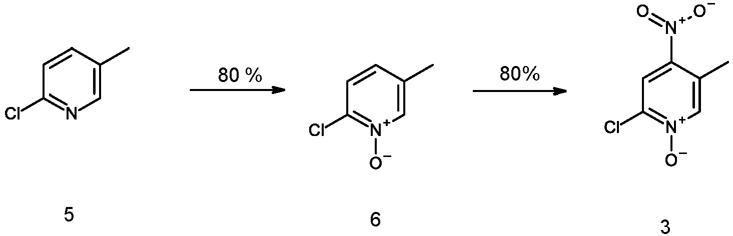

Ein weiterer Gegenstand der vorliegenden Erfindung betrifft ein Verfahren zur Herstellung des Verfahrensintermediats 4-Amino-5-methylpyridon der Formel (I) dadurch gekennzeichnet, dass man zuerst das Intermediat Nitro-N-Oxid (3) an einem Platinkatalysator hydriert und anschließend das erhaltene Intermediat Chlor-Methyl-Amino-Pyridin (2) im Autoklaven mit Kaliumhydroxid (KOH) in Methanol umsetzt.

Eine Ausführungsform betrifft ein Verfahren zur Herstellung des Verfahrensintermediates Chlor-Methyl-Amino-Pyridin (2), indem man Nitro-N-Oxid (3) an einem Platinkatalysator hydriert.

In einer bevorzugten Ausführungsform verwendet man 0,8% Platin (Pt) + 0,6% Molybdän (Mo) auf Carbonpulver als Katalysator verwendet.

In einer weiteren bevorzugten Ausführungsform verwendet man 1 % Platin (Pt) + 2% Vanadium (V) auf Carbonpulver als Katalysator verwendet.

In einer weiteren bevorzugten Ausführungsform verwendet man 0,5 % Platin (Pt) + 0,3 % Molybdän (Mo) auf Carbonpulver als Katalysator verwendet.

Mit dem neuen erfinderischen Verfahren gelingt es ausgehend vom Nitro-N-Oxid (3) über zwei 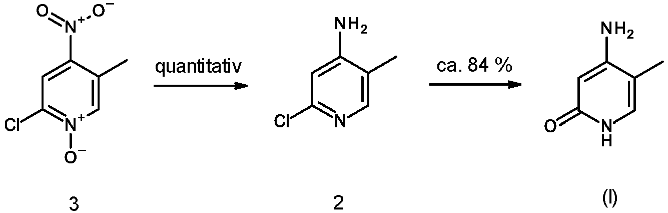 chemische Stufen die Zielverbindung (I) in 84 % Gesamtausbeute mit einer hohen Reinheit (> 99 %) herzustellen. Ein weiterer Vorteil des Verfahrens ist, dass man Verbindung (2) direkt ohne weitere Aufreinigung zu Verbindung (I) umsetzen kann.

Kombiniert man die Ausbeuten des neuen Verfahren mit denen aus der Literatur bekannten, ausgehend vom wohlfeilen und kommerziell sehr günstig zu erhaltenen 2-Chlormethylpyridin (5), so erzielt man eine Gesamtausbeute von 54 % der Theorie das ist eine ca. 2,5 fache Verbesserung der Ausbeute gegenüber des in Synthesis beschriebenen Verfahrens zum Stand der Technik.

Ein weiterer Gegenstand der vorliegenden Erfindung betrifft ein Verfahren zur Herstellung des Verfahrensintermediats 4-Amino-5-methylpyridon der Formel (I) dadurch gekennzeichnet, dass man zuerst das Intermediat Nitro-N-Oxid (3) an einem Platinkatalysator hydriert und anschließend das erhaltene Intermediat Chlor-Methyl-Amino-Pyridin (2) im Autoklaven mit Kaliumhydroxid (KOH) in Methanol umsetzt.

### Beispiele

### Beispiel 1 Herstellung von 2-Chlor-5-methyl-pvridin-4-amin (Verbindung 2)

29 g (153,788 mmol) 2-Chloro-5-methyl-4-nitro-1-oxido-pyridin-1-ium (Verbindung 3, Heterocycles, Vol. 78,No 11, 2009, S 2811) wurden in einem Glasdruckreaktor mit Kreuzbalkenrührer unter Argon vorgelegt und 2,9 g Hydrier-Katalysator (0,8% Pt und 0,6 % Mo auf Aktiv-Kohle (D505A-105 0,8% Pt+ 0,6%Mo on Carbon powder BASF.) und 320 ml Ethanol zugegeben. Der Reaktor wurde verschlossen und 3 mal mit je 3 bar Argon-Überdruck inertisiert. Anschließend wurde 20 Stunden bei 30°C unter 3 bar Wasserstoff-Überdruck hydriert (Umsatz > 98%). Der Reaktor wurde mit Argon inertisiert und die Reaktionslösung über 10 g Kieselgur filtriert. Das Filtrat wurde im Vakuum zur Trockene eingeengt.
Ausbeute: 23,0 g (quantitativ, Produkt enthielt noch Ethanol), Reinheit: 97,5 % (HPLC)
MS (EIpos): m/z = 143 [M+H]+
1H-NMR (300 MHz, DMSO-d6): δ = 1.96 (s, 3H), 6.16 (br s, 2H), 6.50 (s, 1H), 7.68 (s, 1H)

In analoger Weise konnte mit einem Katalysator bestehend aus 0.8 % Pt und 0.3 % Mo auf Aktivkohle ein Umsatz von ca. 98% erzielt werden. Bei Verwendung von 1 % Pt + 2% V auf Aktivkohle konnte ein Umsatz von ca. 87 % erzielt werden.

### Beispiel 2 Herstellung von 4-Amino-5-methyl-1H-pyridin-2-on (I)

4,0 g der Titelverbindung aus Beispiel 1 (Verbindung 2) wurden in 40 ml Methanol in einem Druckreaktor vorgelegt und 12,5 g Kaliumhydroxid (KOH) zugegeben. Dann wurde für 16 Stunden auf 180°C erhitzt (Druckaufbau bis 12,5 bar). Man lässt abkühlen.

Die Reaktion wurde 5-mal mit jeweils 4,0 g der Titelverbindung aus Beispiel 1 durchgeführt und die Reaktionslösungen nach dem Abkühlen vereinigt.

Aufarbeitung: Es wurde unter Kühlung mit ca. 100 ml aqu. 25% Salzsäure auf pH 7,0 gestellt, anschließend wurde unter Vakuum zur Trockene eingeengt und der Rückstand 5-mal mit jeweils 50 ml Ethanol azeotropiert (im Vakuum zur Trockene eingeengt, um Wasserspuren zu entfernen). Zum Eindampfrückstand wurde 400 ml Methanol zugegeben und gerührt. Es wurde vom Salz (KCl) abfiltriert und das Salz zweimal mit 25 ml Methanol gewaschen. Das Filtrat wurde unter Vakuum zur Trockene eingeengt. Der Eindampfrückstand wurde aus 60 ml Wasser um kristallisiert. Man ließ auf 0°C abkühlen und filtrierte die ausgefallen Kristalle ab. Das Feuchtprodukt wurde anschließend unter Vakuum bei 30°C getrocknet.
Ausbeute: 13,5 g (77,53 % der Theorie); Reinheit lt. HPLC: 99,1 %
Aus der Mutterlauge konnten weitere 1,10 g (6,32 % der Theorie) isoliert werden, sodass eine Gesamtausbeute von ca. 84 % der Theorie erreicht wurde
MS (EIpos): m/z = 125 [M+H]+
1H-NMR (300 MHz, DMSO-d6): δ = 1.81 (s, 3H), 2.54 (s, 1H), 5.24 (s, 1H), 5.79 (s, 2H), 6.85 (s, 1H), 10.27 (br s, 1H)

Aus dem oben beschreiben wird deutlich, dass an den bisherigen zur Verfügung stehenden Verfahren, nachteilig ist, dass
(1) eine mehrstufige Synthese durchgeführt wird,
(2) die Nebenprodukte der Formel (VI) (bis > 10%), der Formel (4) und/oder der Formel (7) anfallen, welche als Verunreinigung bei der Herstellung der Verbindung der Formel (I) auftreten und durch aufwendige chromatographische Verfahren abgetrennt werden müssen,
(3) Benzylamin in einem sehr großen Überschuss verwendet wird, dessen Recycling aufwendig und mit erheblichen Kosten verbunden ist,
(4) die Reaktion in siedendem Benzylamin bei 185°C und mit einer Reaktionszeit von 36 Stunden durchgeführt werden muss da solche hohen Temperaturen in Standard-Rührwerken nicht durchführbar sind und erfordern spezielles technisches Equipment,
(5) chlorierte Lösungsmittel eingesetzt werden, die nicht umweltfreundlich sind und
(6) große Mengen Pd-Katalysator auf Kohle eingesetzt werden müssen, dessen Abtrennung und Aufarbeitung nicht nur aufwendig, sondern auch in der großtechnischen Synthese kaum realisierbar sind.

Im Gegensatz dazu werden diese Nachteile durch das erfindungsgemäße Verfahren vermieden und die folgenden Effekte und Vorteile erzielt:
(1) werden weniger Verfahrensschritte oder Synthesestufen benötigt, um zu der Verbindung nach Formel (I) oder zu der Verbindung nach Formel (2) zu gelangen,
(2) die Verbindung nach der Formel (I) wird direkt, ohne Aufreinigung, in hoher Reinheit gewonnen,
(3) die Verbindungen der Formeln (VI), (4) und/oder (7) fallen nicht als unerwünschte Nebenprodukte an,
(4) chromatographische Aufreinigung, wie es im Stand der Technik beschrieben ist, ist nicht erforderlich und macht daher dieses neue erfinderische Verfahren hinsichtlich eines Up-Scalings für die Groß-Produktion sehr attraktiv,
(5) es kann auf den mehrfachen Einsatz von Lösungsmitteln, insbesondere chlorierten Lösungsmitteln, ganz oder teilweise verzichtet werden, so dass das erfindungsgemäße Verfahren deutlich umweltfreundlicher ist und
(6) deutlich geringere Reaktionszeiten und/oder Reaktionstemperaturen erfordert.

Insgesamt stellt das erfindungsgemäße Verfahren eine sehr effiziente, kürzere Synthese, ohne Verwendung von Chromatographie dar, dass auch für ein Up-Scaling geeignet ist. Mit dem erfindungsgemäßen Verfahren gelang es ausgehend vom Nitro-N-Oxid (3) über zwei chemische Stufen die Zielverbindung (I) mit einer Gesamtausbeute von 84 % in einer hohen Reinheit (> 99 %) herzustellen.

## Patentansprüche

1. Verfahren zur Herstellung des Verfahrensintermediats 4-Amino-5-methylpyridon der Formel (I) 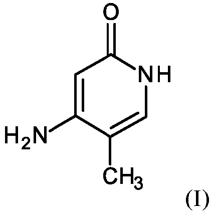 wobei Chlor-Methyl-Amino-Pyridin (2) 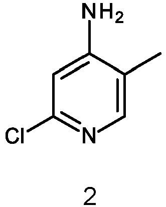 im Autoklaven mit KOH in Methanol umgesetzt wird.

2. Verfahren nach Anspruch 1, wobei die Reaktion bei einer Temperatur im Bereich von 160°C bis 200°C erfolgt.

3. Verfahren nach Anspruch 1 oder 2, wobei die Reaktion bei einer Temperatur von 180°C erfolgt.

4. Verfahren zur Herstellung des Verfahrensintermediats 4-Amino-5-methylpyridon der Formel (I), wobei das Verfahren die folgenden Schritte a) und b) umfasst:
a) Hydrierung des Nitro-N-Oxids der Formel (3) 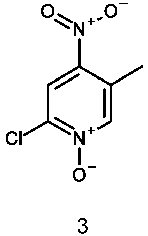 an einem Platinkatalysator, wobei Chlor-Methyl-Amino-Pyridin der Formel (2) erhalten wird, und
b) anschließendes Umsetzen des erhaltenen Intermediats Chlor-Methyl-Amino-Pyridin der Formel (2) im Autoklaven mit KOH in Methanol nach einem der Ansprüche 1 bis 3.

5. Verfahren nach Anspruch 4, wobei in Schritt a) 0,8% Platin und 0,6% Molybdän auf Carbonpulver als Platinkatalysator eingesetzt wird.

6. Verfahren nach Anspruch 4, wobei in Schritt a) 1 % Platin und 2% Vanadium auf Carbonpulver als Platinkatalysator eingesetzt wird.

7. Verfahren nach Anspruch 4, wobei in Schritt a) 0,5 % Platin und 0,3 % Molybdän auf Carbonpulver als Platinkatalysator eingesetzt wird.

8. Verfahren nach einem der Ansprüche 4 bis 7, wobei in Schritt b) die Reaktion bei einer Temperatur im Bereich von 160°C bis 200°C erfolgt.

9. Verfahren nach einem der Ansprüche 4 bis 8, wobei in Schritt b) die Reaktion bei einer Temperatur von 180°C erfolgt.

## Claims

1. Process for preparing the process intermediate 4-amino-5-methylpyridone of formula (I) 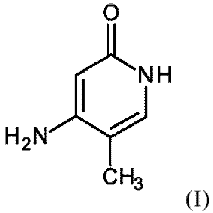 wherein chloro-methyl-aminopyridine (2) 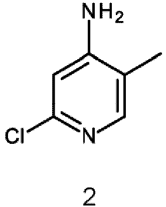 is reacted with KOH in methanol in an autoclave.

2. Process according to Claim 1, wherein the reaction is carried out at a temperature in the range from 160°C to 200°C.

3. Process according to Claim 1 or 2, wherein the reaction is carried out at a temperature of 180°C.

4. Process for preparing the process intermediate 4-amino-5-methylpyridone of formula (I), wherein the process comprises the following steps a) and b):
a) hydrogenation of the nitro-N-oxide of formula (3) 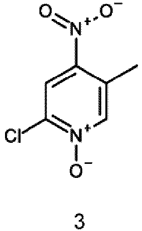 on a platinum catalyst, with chloro-methyl-aminopyridine of formula (2) being obtained, and
b) subsequent reaction of the obtained intermediate chloro-methyl-aminopyridine of formula (2) with KOH in methanol in an autoclave according to any of Claims 1 to 3.

5. Process according to Claim 4, wherein 0.8% platinum and 0.6% molybdenum on carbon powder is used as platinum catalyst in step a).

6. Process according to Claim 4, wherein 1% platinum and 2% vanadium on carbon powder is used as platinum catalyst in step a).

7. Process according to Claim 4, wherein 0.5% platinum and 0.3% molybdenum on carbon powder is used as platinum catalyst in step a).

8. Process according to any of Claims 4 to 7,\ wherein in step b) the reaction is carried out at a temperature in the range from 160°C to 200°C.

9. Process according to any of Claims 4 to 8, wherein in step b) the reaction is carried out at a temperature of 180°C.

## Revendications

1. Procédé de préparation de l'intermédiaire de procédé 4-amino-5-méthylpyridone de formule (I) 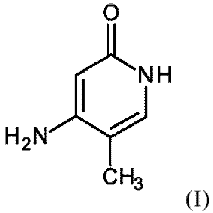 de la chloro-méthyl-amino-pyridine (2) 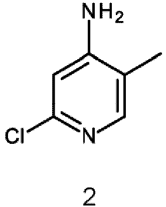 étant transformée avec du KOH dans un autoclave en méthanol.

2. Procédé selon la revendication 1, la réaction ayant lieu à une température dans la plage de 160°C à 200°C.

3. Procédé selon la revendication 1 ou 2, la réaction ayant lieu à une température de 180°C.

4. Procédé de préparation de l'intermédiaire de procédé 4-amino-5-méthylpyrrolidone de formule (I), le procédé comprenant les étapes a) et b) suivantes :
a) hydrogénation du nitro-N-oxyde de formule (3) 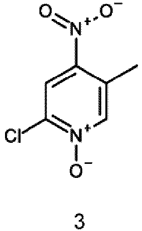 sur un catalyseur à base de platine, de la chloro-méthyl-amino-pyridine de formule (2) étant obtenue et
b) transformation consécutive de l'intermédiaire chloro-méthyl-amino-pyridine de formule (2) obtenu avec du KOH dans un autoclave en méthanol selon l'une des revendications 1 à 3.

5. Procédé selon la revendication 4, 0,8% de platine et 0,6% de molybdène sur de la poudre de carbone étant utilisés dans l'étape a) en tant que catalyseur à base de platine.

6. Procédé selon la revendication 4, 1% de platine et 2% de vanadium sur de la poudre de carbone étant utilisés dans l'étape a) en tant que catalyseur à base de platine.

7. Procédé selon la revendication 4, 0,5% de platine et 0,3% de molybdène sur de la poudre de carbone étant utilisés dans l'étape a) en tant que catalyseur à base de platine.

8. Procédé selon l'une des revendications 4 à 7, la réaction dans l'étape b) ayant lieu à une température dans la plage de 160°C à 200°C.

9. Procédé selon l'une des revendications 4 à 8, la réaction dans l'étape b) ayant lieu à une température de 180°C.
